# EUROPEAN PATENT SPECIFICATION

(11) **EP 2 272 543 B1**
(45) Date of publication and mention of the grant of the patent: **13.04.2016**
(21) Application number: 10011801.7
(22) Date of filing: 20.12.2005
(51) Int. Cl.: A61L 27/28, B29C 53/66, A61L 31/14

(54) **Porous menbranes for medical implants and methods of manufacture**
Poröse Membranen für medizinische Implantate und Verfahren zu ihrer Herstellung
Membranes poreuses pour implants médicaux et méthodes de manufacture

(30) Priority: 20.12.2004 US 637495 P
(43) Date of publication of application: 12.01.2011
(62) Divisional of application: 05821580.7
(73) Proprietor: Abbott Laboratories Vascular Enterprises Limited, Dublin 2 (IE)
(72) Inventor: Fierens, Joost, 1653 Dworp (BE); Huesler, Erhard, 8180 Bülach (DE); Zucker, Arik, 8003 Zürich (CH); Marcoux, Eric, 7190 Ecaussinnes (BE); Nicaise, Philippe, 1180 Uccle (BE); Dubois, Sébastien, 7170 Bois d'Haine (BE)
(74) Representative: Peters, Hajo

(56) References cited:
- US-A- 4 475 972
- US-A- 4 738 740
- US-A- 5 653 747
- US-A1- 2002 113 331
- US-A1- 2002 169 499
- US-A1- 2004 051 201
- US-A1- 2004 126 405
- US-B1- 6 168 409
- Ruediger Landers ET AL: "Desktop manufacturing of complex objects, prototypes and biomedical scaffolds by means of computer assisted design combined with computer-guided 3D plotting of polymers and reactive oligomers", Macromol. mater. Eng, 19 October 2000 (2000-10-19), pages 19-21, XP55011295, DOI: 10.1002/1439-2054(20001001)282:1<17::AID-M AME17>3.0.CO;2-8 Retrieved from the Internet: URL:http://onlinelibrary.wiley.com/doi/10. 1002/1439-2054%2820001001%29282:1%3C17::AI D-MAME17%3E3.0.CO;2-8/abstract?systemMessa ge=Wiley+Online+Library+will+be+disrupted+ 5+Nov+from+10-12+GMT+for+monthly+maintenan ce [retrieved on 2011-11-04]

## Description

### Field Of The Invention

The present invention relates to porous membranes suitable for covering medical implants such as stents for intravascular delivery, implants covered with such membranes and methods for making the porous membranes.

### Background of the Invention

Covered stents for implantation into a body vessel, duct or lumen generally include a stent and a cover attached to the stent. A porous structure of the cover, depending on the porosity, may enhance tissue ingrowth after the covered stent has been implanted. A porous structure affixed to an implantable device also may serve as a reservoir for bioactive components and/or reduce embolization by trapping thrombus against a vessel wall.

Porous membranes for use in medical devices are known in the art. For example, U.S. Patent No. 4,759,757 to Pinchuk describes the formation of a porous membrane by leaching water soluble inorganic salts incorporated into the membrane to create pores where the salt crystals were initially located. U.S. Patent 6,540,776 to Sanders Millare et al. describes a perforated membrane in which a pattern of interstices is created by removing material, for example, by laser cutting. The foregoing manufacturing methods require at least two process steps to form a porous membrane.

One step processes for forming porous membranes also are known in the art, for example, using spinning techniques. U.S. Patent Application Publication No. 20040051201 to Greenhalgh et al. describes an electrospinning process in which a membrane is formed from a plurality of randomly-oriented, intertangled, non-woven fibrils.

Spinning techniques that produce less random, but non-uniform membranes, also are known. For example, U.S. Patent No. 4,475,972 to Wong describes a porous polymeric material made by a process in which polymeric fibers are wound on a mandrel in multiple overlying layers. The fibers contain unevaporated solvent when deposited in contact with one another, so that upon evaporation of the solvent the fibers bond together. The fibers laid in one traverse are wound on the mandrel parallel to each other and at an angle with respect to the axis of the mandrel. In the next traverse, the angle of winding is reverse to the previous angle, so that the fibers crisscross each other in multiple layers to form the porous structure.

U.S. Patent No. 4,738,740 to Pinchuk et al. describes a spinning method similar to that of Wong and further comprising intermittently applying a electrostatic charge to ensure reattachment of broken fibers to the mandrel. U.S. Patent No. 5,653,747 to Dereume describes a vascular graft with an expandable coating produced by the spinning technique of Wong and having pores that open when the tubular support member expands.

US 2004/0126405 discloses a three dimensional cell scaffold which may be used in conjunction with a stent as support member. The scaffold includes a plurality of fibers forming a non-woven three dimensional open celled matrix with a plurality of connections between the fibers having a predetermined pore size and pore shape. All of the foregoing spinning processes suffer from an inability to tightly control the pore size and pore pattern of the resulting membranes. More specifically, lateral deviation of the fibers using previously known spinning techniques has resulted in unsteady collocation of the fibers and the need to deposit multiple layers to ensure adequate coverage. Consequently, previously-known techniques produce either stiff membranes formed of multiple layers and unsatisfactory porosity, or porous, elastic membranes with insufficient strength.

In view of the foregoing, it would be desirable to provide membranes having controlled porosity, pore pattern and pore distribution.

It further would be desirable to provide a one step manufacturing process to produce membranes having controlled porosity, pore pattern and pore distribution.

It still further would be desirable to provide a one step manufacturing process to produce membranes having controlled porosity and/or pore pattern wherein the membrane includes a bioactive substance that may be eluted from the membrane after implantation.

It also would be desirable to provide manufacturing processes to produce membranes having the desired porosity, pattern and distribution characteristics for use in medical implants.

### Summary Of The Invention

In view of the foregoing, it is an object of the present invention to provide membranes for use in medical implants having controlled porosity, pore pattern and pore distribution.

It is another object of this invention to provide a one step manufacturing process to produce membranes having controlled porosity, pore pattern and pore distribution.

It is a further object of the present invention to provide a one step manufacturing process to produce membranes having controlled porosity and/or pore pattern wherein the membrane includes a bioactive substance that may be eluted from the membrane after implantation.

It is also an object of this invention to provide manufacturing processes to produce membranes having the desired porosity, pattern and distribution characteristics for use in medical implants.

These and other objects of the present invention are accomplished by providing a membrane comprising a plurality of fibers that are deposited onto a substrate with a predetermined and reproducible pattern. The substrate is a mandrel
· In a preferred embodiment, the fibers comprise a polymer that is sufficiently elastic and robust that the membrane follows the movements of the stent from.loading onto a stent delivery system to deployment and implantation, without adversely affecting the performance of the membrane of the stent.

In a preferred embodiment, the membrane is formed using a computer-controller substrate that moves in a precisely controlled and reproducible manner. The polymer used to form the fibers, e.g., a polyurethane or a copolymer thereof, is dissolved in a solvent and extruded through one or more extrusion heads onto a moving substrate. By moving the extrusion head back and forth with a specific velocity along the axis of the substrate, specific filament angles or patterns may be deposited. In accordance with one aspect of the present invention, the number of passes, substrate shape and motion and extrusion head speed and material flow are controlled to provide a predetermined fiber diameter that is deposited to produce desired membrane properties, such as pore size and density.

The membrane may either be fixed on the exterior surface of an implantable device, such as a stent, on the interior surface or both.
the covering may be deposited on a mandrel to form a separate component, and then affixed to the implantable device in a later manufacturing step.

In accordance with another aspect of the present invention, the membrane may comprise composite fibers having a viscous sheath co-extruded around a solid core component, or alternatively may comprise co-extruded viscous components. In this manner, a membrane may be created wherein the individual fibers are loaded with a desired bioactive agent, such as a drug, that elutes from the matrix of the membrane without resulting in substantial degradation of the mechanical properties of the membrane.

Methods of manufacturing covered implantable medical devices including the porous membranes of the present invention also are provided.

### Brief Description Of The Drawings

Further features of the invention, its nature and various advantages will be more apparent from the accompanying drawings and the following detailed description of the preferred embodiments, in which:
FIG. 1 is a schematic depiction of a membrane manufacturing system constructed in accordance with the principles of the present invention;
FIGS. 2A-2C are perspective views depicting exemplary patterns for depositing fibers onto a moving substrate in accordance with the present invention;
FIG. 3 is a perspective view illustrating a stent covered with the membrane of the present invention;
FIG. 4 is a schematic depiction of a membrane manufacturing process wherein the fibers comprise a core filament having a polymeric sheath; and
FIG. 5 is a schematic depiction of a membrane manufacturing process wherein the fibers comprise a coextrusion of two polymers.

### Detailed Description Of The Invention

The present invention generally relates to medical implants, such as stents, having a porous membrane and the methods of making such membranes and medical implants. In accordance with the present invention, polymer membranes are provided that have well-defined pores based on a controlled deposition of fibers onto a substrate. In this manner a permeable membrane having a predetermined pore size and distribution may be obtained.

Acute as well as late embolization are a significant threat during and after intravascular interventions such as stenting in saphenous vein grafts (SVG) and carotid arteries, where released particles can lead to major cardiac attacks or strokes, respectively. Covered stents for treatment of atherosclerotic lesions constructed according to the present invention comprise a porous membrane bonded to an exterior surface, and interior surface, or both, of a stent. Advantageously, the covered stent of the present invention may serve both to reduce embolization during an interventional procedure and prevent late embolization by tethering emboli and particles to the vessel wall.

The inventive membrane may be engineered to provide any of a number of design properties, including: single and multi-component material composition; loading of one or more physiological (bioactive) substances into the polymer matrix; predetermined isotropic or an-isotropic mechanical properties; and predetermined pore geometry.

In accordance with the principles of the present invention, polymeric material is deposited onto a computer-controlled movable substrate. Controlling the relative location and motion of the material source with regard to the deposition location on the substrate and process parameters, such as material flow and viscosity of the deposited material, permits generation of a multitude of different patterns for the membrane.

The porous membrane of the present invention is sufficiently strong and flexible for use in medical devices, and preferably comprises steps of extruding a continuous fiber-forming biocompatible polymeric material through a reciprocating extrusion head onto a substrate to form an elongated fiber. The fiber is deposited on the substrate in a predetermined pattern in traces having a width of from 5 to 500 micrometers, adjacent traces being spaced apart from each other a distance of between 0 and 500 micrometers.

Preferably, the fibers have a predetermined viscous creep that allows adjacent traces to bond to one another at predetermined contact points upon deposition. The number of overlapping or crossing fibers generally should be less than 5, preferably less than 4, and most preferably 1 or 2. When cured, the biocompatible material provides a stable, porous membrane.

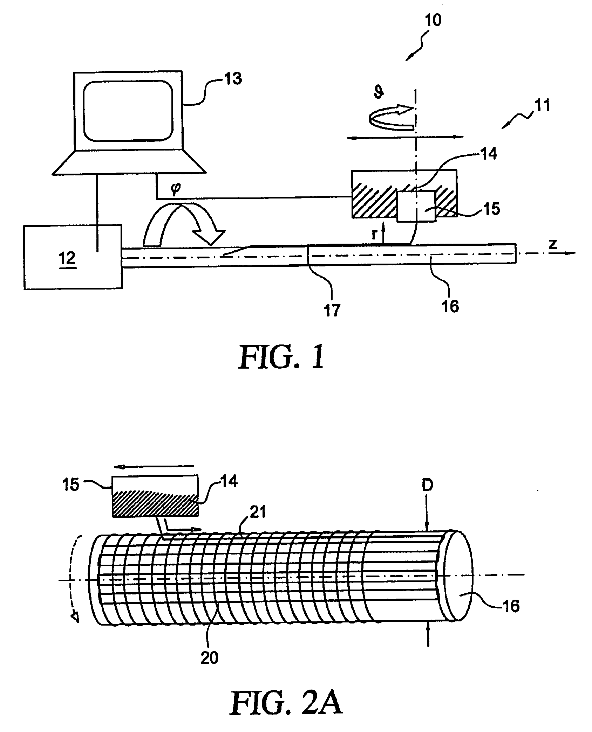

Referring to FIG. 1, apparatus 10 suitable for forming the porous membranes of the present invention comprises polymer extrusion machine 11 coupled to numerically controlled positioning system 12. Computer 13 controls the flow of extrudate 14 through extrusion head 15 as well as relative motion of extrusion head 15 and substrate 16 resulting from actuation of positioning system 12.

Apparatus 10 permits highly-localized deposition of the extrudate with four degrees of freedom onto a substrate to form a membrane. The degrees of freedom are: z - the longitudinal motion of substrate 16 relative to extrusion head 15 ; ϕ - the angular movement of substrate 16 relative to extrusion head 15; r - the distance between extrusion head 15 and substrate 16; and θ - the pivotal angle of extrusion head 15. The polymer strands 17 may be deposited onto the substrate under computer control to form any of the patterns described herein below.

In a preferred embodiment, the substrate comprises a rotating mandrel. Polymer is extruded through reciprocating extrusion head 15 representing the first degree of freedom z, and with a controlled distance between the extrusion head and substrate 16, representing the second degree of freedom r. Preferably, the distance between the extrusion head and substrate is between 0 to 50 mm, and more preferably between 0.5 and 20 mm. As the polymer is deposited onto the substrate, the substrate is rotated through a predetermined angle ϕ, corresponding to the third degree of freedom. In this manner, fibers 17 extruded from extrusion head 15 form a two-dimensional membrane on substrate 16. In addition, by pivoting the extrusion head along its vertical axis, fourth degree of freedom θ may be employed, thus making it possible to deposit more than one filament simultaneously while maintaining a set inter-fiber distance.

The four degrees of freedom discussed above may be independently controlled and if needed, synchronized, to attain a spatial resolution of material deposition having an order of magnitude of microns or higher. Optionally, the second degree of freedom r may be fixed if stable polymer deposition has been achieved. The fourth degree of freedom is not required when extruding only one filament.

Extrusion head 15 may have one or more outlets to deposit an extruded polymer fiber onto substrate 16 in traces having an inter-trace distance ranging between 0 to 1000 micrometers. The width of the individual trace (corresponding to the fiber width) may vary between 5 to 500 micrometers, and more preferably is in the range of 10 to 200 micrometers. Pore size is a function of trace width and inter-trace distance and may be selected by selection of these variables from between 0 (i.e., a tight covering) to 200 micrometers (i.e., to form a filter or tether to trap emboli against a vessel wall). Due to the precise control of fiber deposition, it is possible to create a membrane with desired porosity, strength and flexibility with a very small number of overlapping traces or crossing traces. The number of overlapping or crossing traces in the membrane of the present invention generally should be less than 5, preferably less than 4, and most preferably 1 to 2.

The biocompatible polymer is liquefied either by dissolving the biocompatible material in solvents or by thermally melting the biocompatible material, or both. The viscosity of the liquefied material determines the viscous creep properties and thus final pore size and inter-pore distance when the material is deposited on the substrate. Preferably, the viscous creep is controlled so that desired geometrical and physical properties are met upon deposition. By controlling the viscosity and amount of the deposited material on the substrate and consequently the viscous creep of the polymer before curing, the specified inter-pore distance, pore width and inter-fiber bonding may be achieved. Alternatively, the substrate may be heated to facilitate relaxation and/or curing of the trace width after deposition on the substrate.

Viscosity also may be controlled by adjustment of the distance r of extrusion head 15 relative to substrate 16, the concentration of the solvent in extrudate 14 and/or the heating temperature, ambient pressure, and extrusion parameters. With the viscous creep of the fibers being appropriately controlled, the traces deposited on the substrate will bond to one another at predetermined contact points upon deposition.

A specified pore size of the membrane may be achieved by, but is not limited to, lateral deposition distance between two adjacent material traces, extrusion parameters, and/or extrusion head outlet diameters and extrusion pressure. The latter two parameters also affect the fiber diameter, thus in combination with the fiber deposition pattern selected, permit selection and control of the mechanical properties of the membrane.

Suitable biocompatible materials include but are not limited to polyurethane and copolymers thereof, silicone polyurethane copolymer, polypropylene and copolymers thereof, polyamides, polyethylenes, PET, PEEK, ETFE, CTFE, PTFE and copolymers thereof. Preferred materials for forming membranes of the present invention are polyurethane and copolymers thereof. The polymers may in addition include any biologically active substance having desired release kinetics upon implantation into a patient's body.

Referring now to FIGS. 2A to 2C, exemplary patterns formed by apparatus 10 during deposition of the fibers from extrusion head 15 of the present invention are described. In FIG. 2A, membrane 20 is formed on substrate 16 having diameter D by reciprocating extrusion head 15 longitudinally relative to the longitudinal axis of the substrate, followed by indexed angular movement of the substrate while the extrusion head is held stationary at the ends of the substrate. In this manner, traces 21 having a controlled width and inter-trace spacing may be deposited on the substrate.

Once the longitudinal fibers have been deposited on the substrate, the substrate is rotated 360° while the extrusion head is indexed along the length of the substrate, thereby forming a regular pattern of square or rectangular pores having a predetermined size. Alternatively, if extrusion head 15 is provided with multiple outlets, multiple parallel fibers may be deposited in a single longitudinal pass.

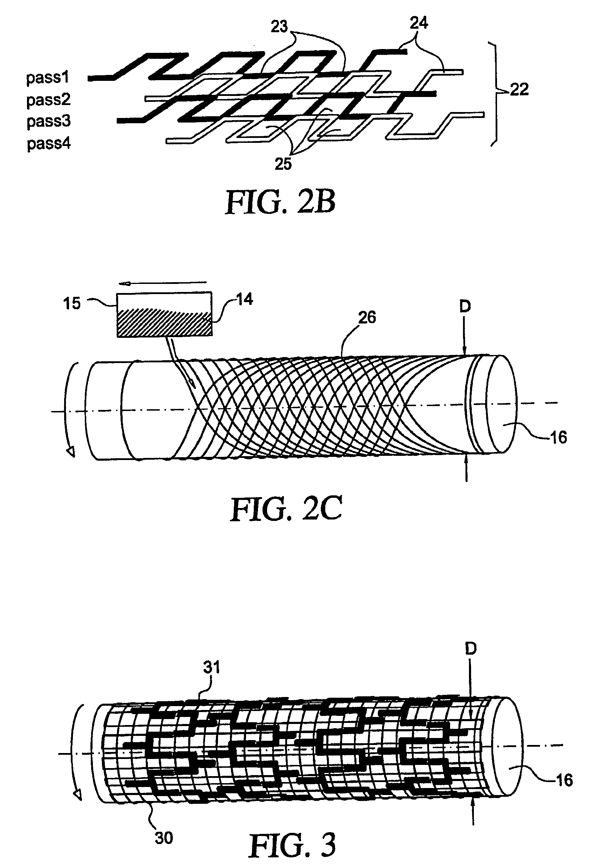

FIG. 2B illustrates alternative membrane pattern 22, wherein the substrate is rotated through precise angular motions during longitudinal translation of the extrusion head. Instead of depositing a straight longitudinal strand, as in the pattern of FIG. 2A, the pattern of FIG. 2B includes a series of "jogs" 23 in each longitudinal filament 24. When adjacent filaments 24 are deposited on the substrate, the contacting portions of the traces bond to one another to define pores 25 having a predetermined size. In this manner, with each longitudinal pass of the extrusion head, a line of pores 25 of predetermined size in formed in a single layer membrane.

FIG. 2C shows another pattern 26 by which the membrane of the present invention may be built up. In this embodiment, positioning system 12 employs two degrees of freedom, z and ϕ, simultaneously, resulting in a "braid-like" structure. Preferably the extruded fibers retain a high unevaporated solvent content when deposited on substrate 16, so that the fibers fuse to form a unitary structure having a predetermined pore size.

More generally, apparatus 10 may be used to deposit one or more traces of a biocompatible material on substrate 16 while extrusion head 15 is reciprocated along the length of the substrate. An extrusion head having multiple outlets permit the deposition of multiple filaments on the substrate during a single translation of the extrusion head or rotation of the substrate. All translational and rotational motions of the components of apparatus 10 are individually or synchronously controlled by computer 13, thus permitting the membrane to be configured with any desired pattern.

As discussed above with respect to FIGS. 2A-2C, apparatus 10 permits fibers to be deposited with any of a number of possible alternative patterns. By depositing the fibers first in multiple passes longitudinal passes followed by indexed translation of the extrusion head and simultaneous rotation of the substrate, as in FIG. 2A, two trace layers may be generated that cross or overlap to form a membrane having a regular grid of pores. In this case, only one degree of freedom is used at any one time. Alternatively, addressing two degrees of freedom alternatingly, as in the pattern of FIG. 2B, a series of "jogs." may be introduced into the individual fibers. In this case, the traces do not cross but only contact each other, thereby creating a line of pores in a single layer membrane. Still further, by addressing two degrees of freedom simultaneously, a braided structure such as depicted in FIG. 2C may be obtained, in which a specified pore size and shape is attained by varying the distance between two parallel traces of material.

In accordance with one aspect of the present invention, extrusion is performed with chemically or thermally liquefied material, or both. The viscosity of the extrudate may be controlled by the concentration of the solvent, by enhancing evaporation of the solvent from the deposited material trace by means of heating the substrate, by varying the distance r between the extrusion head and the substrate, or by adjusting the extrusion temperature of the material so that a well-defined viscous creep of the material occurs after deposition onto the substrate.

Adjustment of the viscous creep allows fusion of the traces at contact points and thus formation of a two-dimensional membrane having desired mechanical strength characteristics. By appropriately setting these parameters accurate material deposition may be achieved with reduced lateral aberrations of the filaments compared to previously-known membrane manufacturing techniqures.

As will of course be understood, the diameter of the substrate should be selected based upon the dimensions of the medical implant or stent to which the membrane is to be affixed. For example, the diameter may be selected based upon the expanded configuration of the medical implant or stent. The implant to be covered may be balloon-expandable or self-expandable. In a preferred embodiment, the implant is a self-expandable stent comprising a superelastic material such as a nickel-titanium alloy.

Referring to FIG. 3, stent 30 covered with membrane 31 of the present invention is described. Stent 30 may comprise any suitable design, such as a plastically deformable slotted tube or self-expanding superelastic structure.
the membrane may be deposited on a mandrel and after curing may be bonded in a separate step to the medical implant. In the latter case, thermal drying and/or evaporation of the solvent cures the biocompatible material while on the substrate. Once the membrane has cured sufficiently so that the mechanical properties of the membrane permit it to be removed from the substrate, the membrane may be bonded to a surface of the implant using a solvent, adhesive or thermal technique. In this case, the surface of the implant may be pre-processed to optimize bonding, for example by activation of the surface, coating of the surface with liquified polymer or other appropriate treatments of the surface.

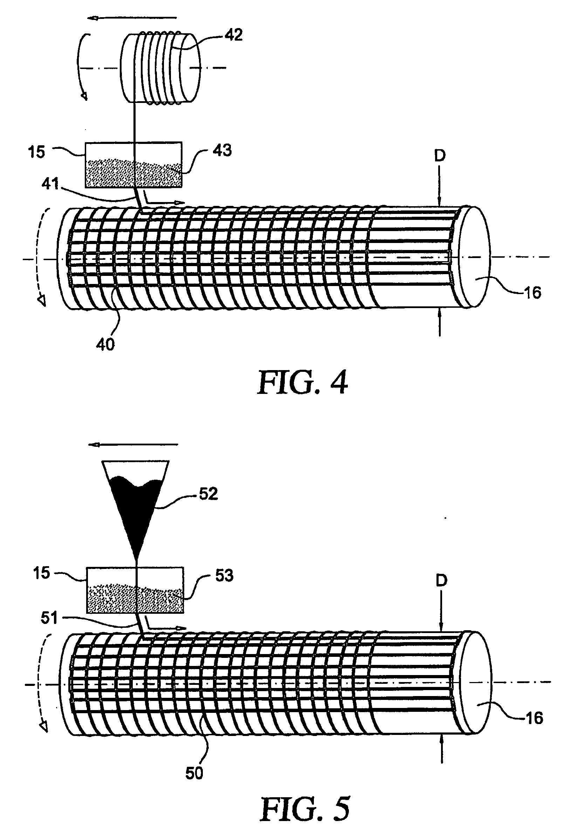

Referring now to FIG. 4, an alternative method of forming a porous membrane, suitable for use in a medical implant, is described. In this embodiment, membrane 40 comprises multi-component fiber 41 including core filament 42 coated with at least second biocompatible material 43 having the same or different chemical, physical, mechanical, biocompatible and or biologically active properties. Material 43 may incorporate one or more biologically active substances that elute into the patient's bloodstream after the medical implant is implanted.

Multi-component fiber 41 may be deposited onto the substrate to form a two-dimensional contiguous structure. The individual components of fiber 41 may be selected to provide different characteristics to the membrane, which may employ any of the pattern designs discussed herein above. For example, core filament 42 may provide mechanical stability, while material 43 may serve as and interface to the biological environment, enhance the adhesive properties for inter-trace bonding and/or enhance bonding of the membrane to the medical implant.

Suitable materials for the core filament include but are not limited to polyamides, polyethylenes, PET, PEEK, ETFE, CTFE, and PTFE and copolymers thereof, and metal wire or fiber glass. Suitable materials for ensheathing core filament 41 include but are not limited to polyurethane and copolymers thereof, silicone polyurethane copolymer, polypropylene and copolymers thereof, polyamides, polyethylenes, PET, PEEK, ETFE, CTFE, PTFE and copolymers thereof.

Referring to FIG. 5, a further alternative method of forming the porous membrane of the present invention is described. In the method depicted in FIG. 5, membrane 50 comprises co-extruded fibers 51 formed of at least first biocompatible material 52 and second biocompatible material 53. Materials 52 and 53 may have the same or different chemical, physical, mechanical, biocompatible and or physiologically active properties. Fibers 51, while illustrated as being co-axially co-extruded, alternatively may be co-extruded co-linearly.

Suitable materials for first material 52 include but are not limited to polyamides, polyethylenes, PET, PEEK, ETFE, CTFE, PTFE and copolymers thereof. Suitable materials for second material 53 include but are not limited to polyurethane and copolymers thereof, silicone polyurethane copolymer, polypropylene and copolymers thereof, polyamides, polyethylenes, PET, PEEK, ETFE, CTFE, PTFE and copolymers thereof.

It should be understood that the present invention is not limited to membranes for use on stents. Rather, the membranes of the present invention may be affixed to any other medical device or implant that is brought into an intracorporal lumen for limited or extended implant durations. Such devices include vascular protection devices to filter emboli that are only transiently introduced into the body. Further applications for such porous membranes may be devices configured to be introduced into other body lumens or ducts, such as the trachea, esophagus, and biliary or urinary lumina.

While preferred illustrative embodiments of the invention are described above, it will be apparent to one skilled in the art that various changes and modifications may be made therein without departing from the invention.
Further embodiments of the invention relate to:
1. A method of making a porous membrane for use in medical implants comprising:
   extruding a continuous fiber-forming biocompatible polymeric material through a reciprocating extrusion head to form an elongated fiber;
   depositing the fiber onto a substrate in.traces having a predetermined pattern and a trace width of 5 to 500 micrometers, adjacent traces being spaced apart a distance of between 0 and 500 micrometers, the fiber having a predetermined viscous creep characteristic that enables the adjacent traces to bond to each other at predetermined contact points; and
   curing the biocompatible material on the substrate to provide a stable, porous membrane.
2. The method defined under 1 wherein depositing the fiber comprises depositing the fiber so that less than five adjacent traces of the fiber overlap or cross.
3. The method defined under 1 wherein depositing the fiber comprises depositing the fiber so that adjacent traces of the fiber do not overlap or cross.
4. The method defined under 3 wherein depositing the fiber comprises depositing the fiber so that adjacent traces of the fiber contact each other only at bond areas to define a row of pores.
5. The method defined under 1, 2, 3 or 4 wherein depositing the fiber comprises depositing the fiber with a high unevaporated solvent content so that adjacent traces of the fiber bond to each other.
6. The method defined under 1 to 5 further comprising providing a substrate, wherein the substrate comprises a vascular implant.
7. The method defined under 6 further comprising providing a medical implant comprising a stent.
8. The method defined under 1 to 7 wherein extruding a continuous fiber-forming biocompatible polymeric material comprises co-extruding a polymeric sheath surrounding a solid core filament.
9. The method defined under 1 to 7 wherein extruding a continuous fiber-forming biocompatible polymeric material comprises co-extruding a first polymeric sheath surrounding a second polymeric core filament.
10. The method defined under 1 to 8 further comprising removing the porous membrane from the substrate and affixing the porous membrane to a surface of a medical implant.
11. An apparatus for making a porous membrane for use in medical implants, the apparatus comprising: an extrusion head having an outlet for extruding a fiber comprising a biocompatible polymer; a substrate; a numerically-controlled positioning system configured to move the extrusion head relative to the substrate, the positioning system providing four degrees of freedom of movement of the extrusion head relative to the substrate; and a computer coupled to control operation of the extrusion head and the positioning system.
12. The apparatus defined under 11 wherein a first degree of freedom comprises translational motion of the extrusion head relative to a longitudinal axis of the substrate.
13. The apparatus defined under 11 or 12 wherein a second degree of freedom comprises rotational motion of the extrusion head relative to a longitudinal axis of the substrate.
14. The apparatus defined under 11, 12 or 13 wherein a third degree of freedom comprises varying a radial distance between the extrusion head and the substrate.
15. The apparatus defined under 11, 12, 13 or 14 wherein a fourth degree of freedom comprises rotating the extrusion head relative to a vertical axis of the extrusion head.
16. The apparatus defined under 11 to 15 further comprising programming that controls the positioning system to rotate the substrate only when the extrusion head is stationary near a proximal or distal end of the substrate.
17. The apparatus defined under 11 to 15 further comprising programming that controls the positioning system to rotate the substrate in alternating directions when the extrusion head is disposed stationary at locations between a proximal end and a distal end of the substrate.
18. The apparatus defined under 11 to 17 further comprising programming that controls the positioning system to vary two or more degrees of freedom simultaneously.
19. The apparatus defined under 11 to 18 wherein the extrusion head is configured to extrude a fiber comprising a first polymer sheath co-extruded surrounding a core filament.
20. The apparatus defined under 11 to 19 wherein the extrusion head is configured to co- linearly extrude multiple fibers.

## Claims

1. A method of making a porous membrane for use in medical implants comprising:
extruding a continuous fiber-forming biocompatible polymeric material through a reciprocating extrusion head to form an elongated fiber;
depositing the fiber onto a substrate in traces having a predetermined pattern by means of using the extrusion head having three or four degrees of freedom of movement relative to the substrate, wherein as the polymeric material is deposited onto the substrate, the substrate is rotated a predetermined angle (ϕ), and the traces having a trace width of 5 to 500 micrometers, adjacent traces being spaced apart a distance of between 0 and 500 micrometers, the fiber having a predetermined viscous creep characteristic that enables the adjacent traces to bond to each other at predetermined contact points, and wherein the substrate comprises a mandrel; and
curing the biocompatible material on the substrate to provide a stable, porous membrane.

2. The method of claim 1 wherein depositing the fiber comprises depositing the fiber so that less than five adjacent traces of the fiber overlap or cross;
or
wherein depositing the fiber comprises depositing the fiber so that adjacent traces of the fiber do not overlap or cross, preferably wherein depositing the fiber comprises depositing the fiber so that adjacent traces of the fiber contact each other only at bond areas to define a row of pores.

3. The method of any one of the preceding claims, wherein depositing the fiber comprises depositing the fiber with a high unevaporated solvent content so that adjacent traces of the fiber bond to each other,
and/or
further comprising providing a substrate, wherein the substrate comprises a vascular implant; preferably further comprising providing a medical implant comprising a stent.

4. The method of any one of the preceding claims, wherein extruding a continuous fiber-forming biocompatible polymeric material comprises co-extruding a polymeric sheath surrounding a solid core filament.
or
wherein extruding a continuous fiber-forming biocompatible polymeric material comprises co-extruding a first polymeric sheath surrounding a second polymeric core filament.

5. The method of any one of claims 1 to 4 further comprising removing the porous membrane from the substrate and affixing the porous membrane to a surface of a medical implant.

6. Apparatus for making a porous membrane for use in medical implants, the apparatus comprising:
an extrusion head having an outlet for extruding a fiber comprising a biocompatible polymer;
a substrate comprising a mandrel;
a numerically-controlled positioning system configured to move the extrusion head relative to the substrate, the positioning system providing three or four degrees of freedom of movement of the extrusion head relative to the substrate wherein a first degree of freedom comprises rotational motion of the extrusion head relative to a longitudinal axis of the substrate such that the apparatus is adapted to rotate the substrate a predetermined angle as the polymer is deposited onto the substrate; and
a computer coupled to control operation of the extrusion head and the positioning system.

7. The apparatus of claim 6 wherein a second degree of freedom comprises translational motion of the extrusion head relative to a longitudinal axis of the substrate.

8. The apparatus of claim 6 or 7 wherein a third degree of freedom comprises varying a radial distance between the extrusion head and the substrate;
and/or
wherein a fourth degree of freedom comprises rotating the extrusion head relative to a vertical axis of the extrusion head.

9. The apparatus of any one of claims 6 to 8, wherein the apparatus is adapted for programming that controls the positioning system to rotate the substrate only when the extrusion head is stationary near a proximal or distal end of the substrate;
or
the apparatus further being adapted for programming that controls the positioning system to rotate the substrate in alternating directions when the extrusion head is disposed stationary at locations between a proximal end and a distal end of the substrate.

10. The apparatus of any one of claims 6 to 9, the apparatus further being adapted for programming that controls the positioning system to vary two or more degrees of freedom simultaneously;
and/or
wherein the extrusion head is configured to extrude a fiber comprising a first polymer sheath co-extruded surrounding a core filament.
and/or
wherein the extrusion head is configured to co- linearly extrude multiple fibers.

## Patentansprüche

1. Ein Verfahren zur Herstellung einer durchlässigen Membran zur Verwendung in medizinischen Implantaten, umfassend:
Extrudieren eines fortlaufenden, faserbildenden biokompatiblen Polymers durch einen hin- und hergehenden Extrusionskopf, um eine längliche Faser zu bilden;
Aufbringen der Faser auf Spuren eines Substrats mit einem festgelegtem Muster, wobei der Extrusionskopf drei oder vier Freiheitsgrade der Bewegung relativ zu dem Substrat hat, wobei das Substrat, wenn das Polymer auf das Substrat aufgebracht wird, um einen festgelegten Winkel (ϕ) gedreht wird, und die Spuren eine Spurweite von 5 bis 500 Mikrometer haben, wobei nebeneinander liegende Spuren zwischen 0 und 500 Mikrometer voneinander entfernt liegen, wobei die Faser eine vorgegebene viskose Zähigkeit aufweist, welche es ermöglicht, dass sich nebeneinander liegende Spuren an festgelegten Kontaktpunkten miteinander verbinden und wobei das Substrat eine Spindel aufweist; und
Aushärten des biokompatiblen Materials auf dem Substrat, um eine stabile, poröse Membran bereitzustellen.

2. Das Verfahren nach Anspruch 1, wobei das Aufbringen des Substrats so erfolgt, dass weniger als fünf nebeneinander liegende Spuren der Faser überlappen oder sich kreuzen;
oder
wobei das Aufbringen des Substrats Aufbringen der Faser derart umfasst, dass nebeneinander liegende Spuren der Faser nicht überlappen oder sich kreuzen, wobei vorzugsweise das Aufbringen der Faser umfasst, die Faser derart aufzubringen, dass nebeneinander liegende Spuren der Faser sich nur an Verbindungsstellen verbinden um eine Reihe von Poren zu definieren.

3. Das Verfahren nach einem der vorstehenden Ansprüche, wobei das Aufbringen der Faser ein Aufbringen der Faser mit einem hohen Anteil an unverdampftem Lösungsmittel umfasst, sodass sich nebeneinander liegende Spuren der Faser miteinander verbinden,
und/oder
ferner umfassend Bereitstellen eines Substrats, wobei das Substrat ein vaskuläres Implantat umfasst, vorzugsweise außerdem ein medizinisches Implantat bereitzustellen, das einen Stent umfasst.

4. Das Verfahren nach einem der vorstehenden Ansprüche, wobei das Extrudieren eines fortlaufenden, faserbildenden biokompatiblen Polymers das Ko-Extrudieren einer Polymer-Umhüllung, die eine feste Kernfaser umschließt umfasst;
oder
wobei das Extrudieren eines fortlaufenden, faserbildenden biokompatiblen Polymers das Ko-Extrudieren einer ersten Polymer-Umhüllung umfasst, die eine zweite Polymer-Kernfaser umschließt.

5. Das Verfahren nach einem der Ansprüche 1 bis 4, ferner umfassend Entfernen der porösen Membran von dem Substrat und Befestigen der porösen Membran an einer Oberfläche eines medizinischen Implantats.

6. Eine Vorrichtung zur Herstellung einer porösen Membran zur Verwendung in medizinischen Implantaten, wobei die Vorrichtung umfasst:
einen Extrusionskopf mit einem Ausgang zum Extrudieren einer Faser, welche ein biokompatibles Polymer umfasst;
ein Substrat, das eine Spindel umfasst;
ein numerisch gesteuertes Positionierungssystem, das ausgelegt ist, um den Extrusionskopf relativ zum Substrat zu bewegen, wobei der Extrusionskopf durch das Positionierungssystem drei oder vier Freiheitsgrade der Bewegung relativ zu dem Substrat hat, wobei ein erster Freiheitsgrad eine Drehbewegung des Extrusionskopfes relativ zu einer Längsachse des Substrats umfasst, sodass die Vorrichtung ausgelegt ist, um das Substrat um einen festgelegten Winkel zu drehen, wenn das Polymer auf das Substrat aufgebracht wird; und
einen gekoppelten Computer um den Betrieb des Extrusionskopfes und des Positionierungssystems zu steuern/regeln.

7. Die Vorrichtung nach Anspruch 6, wobei ein zweiter Freiheitsgrad eine Verschiebebewegung des Extrusionskopfes relativ zu einer Längsachse des Substrats umfasst.

8. Die Vorrichtung nach Anspruch 6 oder 7, wobei ein dritter Freiheitsgrad das Ändern eines radialen Abstandes zwischen dem Extrusionskopf und dem Substrat umfasst;
und/oder
wobei ein vierter Freiheitsgrad das Drehen des Extrusionskopfes relativ zu einer vertikalen Achse des Extrusionskopfes umfasst.

9. Die Vorrichtung nach irgendeinem der Ansprüche 6 bis 8, wobei die Vorrichtung auf eine Programmierung ausgelegt ist, die das Positionierungssystem so steuert, dass das Substrat nur dann gedreht wird, wenn der Extrusionskopf in der Nähe des proximalen oder distalen Endes des Substrats im Ruhezustand ist;
oder
wobei die Vorrichtung ferner auf eine Programmierung ausgelegt ist, die das Positionierungssystem so steuert, dass das Substrat in abwechselnde Richtungen gedreht wird, wenn der Extrusionskopf zwischen einem proximalen und einem distalen Ende des Substrats im Ruhezustand ist.

10. Die Vorrichtung nach irgendeinem der Ansprüche 6 bis 9, wobei die Vorrichtung ferner auf eine Programmierung ausgelegt ist, die das Positionierungssystem so steuert, dass es zwei oder mehr Freiheitsgrade gleichzeitig ändert;
und/oder
wobei der Extrusionskopf so konfiguriert ist, dass er eine Faser extrudiert, die eine erste, ko-extrudierte Polymer-Umhüllung umfasst, die eine Kernfaser umhüllt;
und/oder
wobei der Extrusionskopf so konfiguriert ist, dass mehrere Fasern gleichzeitig linear extrudiert werden.

## Revendications

1. Méthode de production d'une membrane poreuse à usage destiné à des implants médicaux consistant à :
extruder un matériau polymère biocompatible à fibre continue au moyen d'une tête d'extrusion alternative afin de former une fibre allongée ;
déposer la fibre sur un support sous forme d'empreintes à la forme prédéterminée au moyen de la tête d'extrusion ayant trois à quatre degrés de liberté de mouvement par rapport au support, le matériau polymère étant déposé sur le support et le support effectuant une rotation selon un angle prédéfini (ϕ), et la largeur des empreintes étant de 5 à 500 micromètres, les empreintes adjacentes étant séparées de 0 à 500 micromètres, la fibre ayant des caractéristiques de fluage prédéfinies permettant aux empreintes adjacentes de se lier les unes aux autres à des points de contacts prédéterminés; le support comportant un mandrin ; et
durcir le matériau biocompatible sur le support afin d'offrir une membrane poreuse stable.

2. Méthode selon la revendication 1, dans laquelle le dépôt de la fibre comprend le croisement ou la superposition de moins de cinq empreintes adjacentes de la fibre :
ou
dans laquelle le dépôt de la fibre comprend que les empreintes adjacentes de la fibre ne se croisent pas ou ne se superposent pas, de préférence dans laquelle le dépôt de la fibre comprend que les empreintes adjacentes de la fibre entrent en contact seulement au niveau des zones de connexion afin de permettre la définition d'une rangée de pores.

3. Méthode selon l'une quelconque des revendications précédentes, dans laquelle le dépôt de la fibre comprend une forte teneur en solvant non évaporé de façon à ce que les empreintes adjacentes de la fibre se lient les unes aux autres,
et/ou
comprend en outre la production d'un support, lequel support comprend un implant vasculaire ; comprenant en outre de préférence un implant médical comprenant un stent.

4. Méthode selon l'une quelconque des revendications précédentes, dans laquelle l'extrusion du matériau polymère biocompatible à fibre continue comprend la coextrusion d'une gaine en polymère entourant un filament de gaine solide.
ou
dans laquelle l'extrusion du matériau polymère biocompatible à fibre continue comprend la coextrusion d'une première gaine en polymère entourant un second filament de gaine polymère.

5. Méthode selon l'une quelconque des revendications 1 à 4 comprenant en outre le retrait de la membrane poreuse du support et la fixation de la membrane poreuse sur la surface d'un implant médical.

6. Dispositif de fabrication d'une membrane poreuse pour usage destiné à des implants médicaux comprenant:
une tête d'extrusion dotée d'une sortie permettant l'extrusion d'une fibre comprenant un polymère biocompatible ;
un support comprenant un mandrin ;
un système de positionnement à commande numérique configuré de façon à déplacer la tête d'extrusion par rapport au support, le système de positionnement comprenant trois à quatre degrés de liberté de mouvement de la tête d'extrusion par rapport au support, le premier degré de liberté comprenant un mouvement rotatif de la tête d'extrusion par rapport à l'axe longitudinal du support, de façon à ce que le dispositif soit adapté à la rotation du support selon un angle prédéfini lorsque le polymère est déposé sur le support ; et
un ordinateur couplé permettant de contrôler le fonctionnement de la tête d'extrusion et du système de positionnement.

7. Dispositif selon la revendication 6, dans lequel un second degré de liberté comprend un mouvement de translation de la tête d'extrusion par rapport à l'axe longitudinal du support.

8. Dispositif selon la revendication 6 ou 7, pour lequel un troisième degré de liberté comprend une distance radiale variable entre la tête d'extrusion et le support ;
et/ou
dans lequel un quatrième degré de liberté comprend la rotation de la tête d'extrusion par rapport à l'axe vertical de la tête d'extrusion.

9. Dispositif de l'une quelconque des revendications 6 à 8, dans lequel le dispositif est adapté pour une programmation qui contrôle le système de positionnement permettant la rotation du support seulement lorsque la tête d'extrusion est stationnaire entre une extrémité proximale et une extrémité distale du support :
ou
le dispositif est ultérieurement adapté pour une programmation qui contrôle le système de positionnement permettant la rotation du support dans des directions alternées lorsque la tête d'extrusion est stationnaire dans des zones situées entre l'extrémité proximale et l'extrémité distale du support.

10. Dispositif selon l'une quelconque des revendications 6 à 9, dans lequel le dispositif est en outre adapté pour une programmation qui contrôle le système de positionnement permettant une variation de deux ou degrés ou plus de liberté simultanément ;
et /ou
dans lequel la tête d'extrusion est configurée de façon à permettre l'extrusion d'une fibre comprenant une première gaine de polymère co-extrudée entourant un filament de gaine ;
et/ou
dans lequel la tête d'extrusion est configurée de façon à permettre une extrusion co-linéaire de fibres multiples.
